(19) Europäisches Patentamt
European Patent Office
Office européen des brevets

(11) EP 0 084 341 B2

(12) NEW EUROPEAN PATENT SPECIFICATION

(45) Date of publication and mention of the opposition decision:
**14.05.1997 Bulletin 1997/20**

(45) Mention of the grant of the patent:
**19.04.1989 Bulletin 1989/16**

(21) Application number: **83100171.4**

(22) Date of filing: **11.01.1983**

(51) Int. Cl.$^6$: **A61K 9/10**, A61K 7/00

# (54) Emulsion-type composition for external use

Mittel vom Emulsion-Typ für den äusserlichen Gebrauch

Composition du type émulsion pour utilisation par voie externe

(84) Designated Contracting States:
**BE CH DE FR GB IT LI NL SE**

(30) Priority: **11.01.1982 JP 1736/82**

(43) Date of publication of application:
**27.07.1983 Bulletin 1983/30**

(73) Proprietor: **Eisai Co., Ltd.**
**Tokyo 112 (JP)**

(72) Inventors:
- **Ohsawa, Shigemitsu**
**Honjo-shi Saitama (JP)**
- **Amada, Iwao**
**Gunma (JP)**

(74) Representative: **Hansen, Bernd, Dr.Dipl.-Chem. et al**
**Hoffmann, Eitle & Partner**
**Patentanwälte**
**Postfach 81 04 20**
**81904 München (DE)**

(56) References cited:
**EP-A- 0 062 896**
**WO-A-81/02673**
**BE-A- 864 450**
**DE-A- 2 928 040**
**DE-A- 2 928 041**
**DE-B- 1 900 961**
**FR-A- 2 452 314**

- **Seifen, Öle, Fette, Wachse, 100 (14), 1979, pp 343-346**
- **Cosmetics & Toiletries, 94, August 1979, pp 25-30**
- **Reinhard von Kleinsorgen : "Herstellung und Stabilisierung von Emulsionen mit Hilfe von Lecithin", Inaugural Dissertation 1979, Marburg/Lahn**
- **Merkblatt "Emulmetik 88" (1975), Lucas Meyer Hamburg**

Remarks:
The file contains technical information submitted after the application was filed and not included in this specification

EP 0 084 341 B2

## Description

The present invention relates to an emulsion-type composition for external use having a high affinity for the skin and a stable emulsion state.

In the production of an emulsion-type composition for external use, synthetic surfactants such as polyoxyethylene adducts have been used for emulsifying an oil or fat or an oil-soluble component and water or a water-soluble component in the composition. However, while they have an excellent emulsifying power, the polyoxyethylene adduct-type surfactants do not always satisfactory impart a mild affinity for the skin to the composition. It has been tried recently to use a basic amino acid salt of a fatty acid as an emulsifying agent in place of such a synthetic surfactant particularly in the field of cosmetics (Japanese Patent Applications Nos. 127131/1980, 127132/1980, 127133/1980 and 97208/1981). However, the compositions thus prepared have an alkaline pH and their affinity for the skin is poor, since the surfactant contained therein has an excessive hydrophilic character.

When natural gums such as acacia, xanthane gum, algae colloid and synthetic, water-soluble polymers such as CMC—Na, HPC and sodium polyacrylate are used in place of the synthetic surfactants, the emulsifying power obtained is inferior.

H. Rebmann, Seifen-Öle-Fette-Wachse 100 (1974), pages 343-346, discloses a composition of lecithin and possibilities of its use in cosmetic preparations.

G. S. Kass, Cosmetics & Toiletries 94 (1979) 25-30 describes new techniques for formulating cosmetics with lecithin.

EP—A—0 062 896, which forms part of the state of the art by virtue only of Article 54(3), discloses a skin care base material for external use which comprises a phospholipid, an ester of a fatty acid and a polyoxyethylene fatty acid ester. In particular, a composition comprising egg oil, glyceryl monostearate and polyoxyethylene monostearate is disclosed.

It has been impossible to obtain an emulsion-type composition for external use having a high affinity for the skin and satisfactory emulsion stability in the prior art. After investigations made for the purpose of obtaining an emulsion-type composition for external use having an excellent affinity for the skin and emulsion stability under these circumstances, the inventors have found that a desired composition can be obtained by incorporating lecithin and a fatty acid monoester of glycerine or propylene glycol.

Subject matter of the present invention is an emulsion-type composition according to claim 1.

Preferred embodiments thereof are subject matter of claims 2 and 3.

The present invention will now be illustrated in detail.

The term "lecithin" herein involves refined or non-refined phospholipid-containing substances extracted from egg yolk or vegetgable oils or hydrogenation products thereof and synthetic glycerophosphates of the following formula and mixtures thereof:

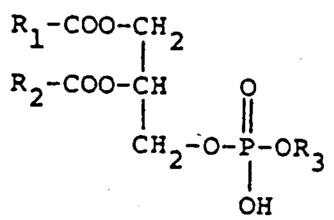

wherein $R_1$ and $R_2$ represent a saturated or unsaturated hydrocarbon having 8 to 20 carbon atoms and $R_3$ represents hydrogen, choline, ethanolamine, serine, inositol or glycerol.

It has been known that lecithin which is one of the constituents of sebum has a high affinity for the skin and is an excellent cosmetic component. However, it is difficult to obtain a stable, emulsion-type composition for external use by using lecithin alone, since the latter has a low emulsifying power. Therefore, to improve the emulsion stability without decreasing the affinity of lecithin for the skin it is necessary to find a special combination of lecithin with a suitable substance.

The fatty acids from which the fatty acid mono-esters are derived are preferably those having 8 to 32 carbon atoms. As the fatty acids, there may be mentioned caprylic acid, capric acid, lauric acid, myristic acid, palmitic acid, stearic acid, behenic acid, oleic acid, linoleic acid, linolenic acid, ricinoleic acid, isostearic acid, hydroxystearic acid, cerotic acid, abietic acid, isooctanoic acid, isohexadecanoic acid, 2-ethyl-hexadecanoic acid and lanolin fatty acids.

Further, mixtures of two or more fatty acid mono-esters of glycerine or propylene glycol may be used as the fatty acid mono-ester of glycerine or propylene glycol in the present invention.

As for the weight ratio of lecithin to the fatty acid mono-ester of glycerine or propylene glycol in the composition of the present invention, 0.2 to 10 parts by weight of the fatty acid mono-ester is used per part by weight of lecithin. It is apparent from Experimental Example 2 given below that if the fatty acid mono-ester is used in an amount of less than 0.05 part by weight, the resulting composition has a poor emulsion stability. It is understood, therefore, that the minimum, necessary amount of the fatty acid mono-ester for attaining an acceptable stability is 0.05 part by weight. If the amount exceeds 20 parts by weight, consistency of the composition *per se* is increased seriously to deteriorate its commercial value, though the emulsion stability is improved. Therefore, it is desirable to control the amount of the fatty acid mono-ester below 20 parts by weight.

The emulsion-type composition of the present invention contains as indispensable constituents an oil, fat or oil-soluble component such as animal oil, vegetable oil, synthetic ester, wax and fat-soluble vitamins and water or water-soluble components and, if necessary, antiseptic, humectant, thickening agent, U.V. absorber, antioxidant and perfume. Cosmetic components such as vitamins, polypeptides and acylated polypeptides may also be incorporated therein. If necessary, the composition may contain surfactants other than those of the present invention such as nonionic surfactants, e.g. polyoxyethylene/fatty acid esters, polyoxyethylene/higher alcohol ethers and polyoxyethylene/sorbitan/fatty acid esters, and anionic surfactants, e.g. higher alcohol/sulfuric acid ester salts, alkali metal salts of the fatty acids and fatty acid/basic amino acid salts (Japanese Patent Publication No. 127318/1980).

The composition of the present invention may be of either oil-in-water type or water-in-oil type as shown in examples given below. The composition of either type may be obtained by suitably selecting starting materials such as lecithin, fatty acid monoester of glycerine or propylene glycol, oil, fat and oil-soluble component, water and water-soluble component and proportions thereof. The composition of the present invention may be in the form of a semi-solid cream or ointment or liquid emulsion. The composition of a desired form can be obtained by suitably selecting the starting materials and proportions thereof. The composition of the present invention may be produced by a general method of producing emulsions. In an embodiment of the method, a mixture of the oil, fat, oil-soluble component, lecithin and fatty acid monoester of glycerine or propylene glycol is heated to 60 to 70°C to obtain a solution, to which are then added water and a water-soluble component heated to the same temperature as above under stirring to obtain an emulsion and the emulsion is cooled to 30 to 40°C. As a matter of course, this method may be modified or varied suitably.

As shown in Experimental Examples given below, the composition of the present invention has a characteristic, high affinity for the skin and emulsion stability. Particularly, the composition has a high affinity for the skin and gives rise to a comfortable feeling upon use. The composition may be used, therefore, for both medical and cosmetic applications. The composition may be used for the production of medicines for external use containing medicinal ingredients such as vitamins or steroid hormons as the active ingredient. Still preferred results can be obtained if the composition is used for the production of cosmetics such as moisturising cream, night cream, massage cream, cleansing cream, lotion and pack.

The following Experimental Examples will further illustrate the effects of the present invention.

Experimental Example 1

Samples:

A specimen example of the present invention and control samples I and II each having a composition shown in the following table were prepared. In the table, the partially hydrogenated egg yolk lecithin had a phospholipid content of 25% and stearic acid/arginine salt comprised 2.5 parts by weight of stearic acid and 0.5 part by weight of ℓ-arginine.

| Components \ Sample | Specimen sample of the invention | Control Sample I | Control Sample II |
|---|---|---|---|
| (1) Squalane | 15.0 parts by wt. | 15.0 parts by wt. | 15.0 parts by wt. |
| (2) Partially hydrogenated egg yolk lecithin | 5.0 | 5.0 | 5.0 |
| (3) Propylene glycol monostearate | 3.0 | — | — |
| (4) Cetyl alcohol | 1.0 | 1.0 | 1.0 |
| (5) Stearic acid | 2.0 | 2.0 | 2.0 |
| (6) Tween® 60 | — | 3.0 | — |
| (7) Stearic acid/arginine salt | — | — | 3.0 |
| (8) Methylparaben | 0.2 | 0.2 | 0.2 |
| (9) Glycerol | 10.0 | 10.0 | 10.0 |
| (10) Purified water | 64.8 | 64.8 | 64.8 |

The samples were prepared by heating components (1) to (7) to obtain a homogeneous solution, maintaining the solution at 75°C, adding a solution comprising constituents (8), (9) and (10) previously prepared by heating to 75°C to the former solution under stirring to effect emulsification and cooling the emulsion to 30°C under stirring.

Method:

40 women were divided into two groups A and B each comprising 20 women. Each sample was used as a moisturising cream continuously for one month. Thereafter, effects thereof were examined with respect to items shown in Table 1 and efficiency was determined according to the following formula:

$$\text{Efficiency (\%)} = \frac{\text{(Number of effective cases)+(Number of slightly effective cases)}}{\text{(Number of effective cases)+(Number of slightly effective cases)+(Number of ineffective cases)}} \times 100$$

Results:

The results are shown in Table 1. It is understood from Table 1 that the composition of the present invention has excellent effects of relieving roughness of the skin and giving moisture to the skin.

Table 1

| Item | Effect | Group A (20 subjects) | | Group B (20 subjects) | |
|---|---|---|---|---|---|
| | | Specimen sample | Control sample I | Specimen sample | Control sample II |
| Effect of relieving roughness of the skin | Effective | 9 | 6 | 11 | 8 |
| | Slightly effective | 10 | 6 | 8 | 9 |
| | Ineffective | 1 | 12 | 1 | 3 |
| | Efficiency | 95% | 60% | 95% | 85% |
| Effect of giving moisture to the skin | Effective | 13 | 8 | 14 | 11 |
| | Slightly effective | 7 | 6 | 6 | 7 |
| | Ineffective | 0 | 6 | 0 | 2 |
| | Efficiency | 100% | 70% | 100% | 90% |
| Skin irritation (smart, itch, etc.) | Recognized | 0 | 1 | 0 | 0 |
| | Slightly recognized | 1 | 1 | 0 | 1 |
| | Not recognized | 19 | 18 | 20 | 19 |

Experimental Example 2

Samples and method:

Samples A to I each having a composition shown in Table 2 were prepared. The partially hydrogenated egg yolk lecithin shown in the table had a phospholipid content of 65%. Each sample was prepared as follows. 5.5 g of component (2) or (3) or combination thereof was added to 15 g of squalane. The mixture was heated to obtain a solution. The solution was maintained at 75°C. Purified water heated to 75°C was added to the solution under stirring to obtain an emulsion. The emulsion was cooled to 35°C under stirring to obtain the sample.

The samples were stored each at room temperature and 40°C and changes in the emulsion state thereof with time were examined.

Table 2

| | Components \ Sample | (I) A | (I) B | (I) C | (I) D | (I) E | (II) F | (II) H |
|---|---|---|---|---|---|---|---|---|
| (1) | Squalane | 15 g | 15 | 15 | 15 | 15 | 15 | 15 |
| (2) | Glycerol monostearate | 5.5 | — | — | — | — | 2.0 | — |
| | Sorbitan monostearate | — | 5.5 | — | — | — | — | — |
| | Propylene glycol monostearate | — | — | 5.5 | — | — | — | 2.5 |
| | Sucrose stearate | — | — | — | 5.5 | — | — | — |
| (3) | Partially hydrogenated egg yolk lecithin | — | — | — | — | 5.5 | 3.5 | 3.0 |
| (4) | Purified water | 79.5 g | 79.5 | 79.5 | 79.5 | 79.5 | 79.5 | 79.5 |

Results:

In samples A to E, a remarkable aqueous phase separation was recognised after only three days at room temperature, while samples F and H were stable and no changes in appearance were recognized even after one month at 40°C. It is understood, therefore, that the combination of lecithin and fatty acid monoester of glycerine or propylene glycol exhibits an excellent emulsifying power, while they exhibit only poor emulsion stabilizing capacity when they are used alone.

Experimental Example 3

Samples:

Samples A to G each having a composition shown in Table 3 were prepared. The partially hydrogenated egg yolk lecithin shown in Table 3 had a phospholipid content of 25%. The amount (part by weight) of glycerol monostearate per

part by weight of the partially hydrogenated egg yolk lecithin in each sample is also shown in the table. Each sample was prepared as follows: components (1) and (2) were added to components of group (3) shown in the table, the mixture was heated to obtain a solution, the solution was maintained at 70°C, a homogeneous solution comprising components (4) previously heated to 70°C was added to form an emulsion, and the emulsion was cooled to 35°C under stirring to obtain the sample.

Table 3

| Components \ Sample | A | B | C | D | E | F | G |
|---|---|---|---|---|---|---|---|
| (1) Partially hydrogenated egg yolk lecithin | 5.0 g | 5.0 g | 5.0 g | 5.0 g | 5.0 g | 5.0 g | 5.0 g |
| (2) Glycerol monostearate | 0 | 0.125 | 0.25 | 0.5 | 1.0 | 2.5 | 5.0 |
| (3) Isopropyl myristate | 5.0 | ditto | ditto | ditto | ditto | ditto | ditto |
| (3) Saturated fatty acid ($C_8$-$C_{12}$) triglyceride | 5.0 | | | | | | |
| (3) Stearic acid | 1.0 | | | | | | |
| (3) Cholesterol | 0.6 | | | | | | |
| (4) Glycerol | 10.0 | | | | | | |
| (4) Methyl p-hydroxybenzoate | 0.2 | | | | | | |
| (4) Purified water | ad 100.0 g | | | | | | |
| Glycerol monostearate (part by weight) | 0.0 | 0.025 | 0.05 | 0.1 | 0.2 | 0.5 | 1.0 |

The samples were stored each at room temperature and 40°C and changes in the emulsion state with time were examined.

Results:

In samples A and B, a remarkable aqueous phase separation was recognized after only three days at room temperature. In sample C, a slight aqueous phase separation was recognized in a lower layer after 10 days at room temperature. However, it had a higher stability than those of samples A and B. In samples E, F and G, the phase separation was not recognized at all and they had a stable appearance even storage not only at room temperature but also at 40°C for one month.

Experimental Example 4

Samples and method:

Samples A to F were prepared. The samples comprised common components (3), (4), (5) and (6) and components (1) and (2) which were various. Components (1) and (2) in the respective emulsions were as shown in Table 4.

Recipe:

| Components | Parts by weight |
|---|---|
| (1) See Table 4 | 25.0 |
| (2) See Table 4 | 6.0 |
| (3) Partially hydrogenated egg yolk lecithin | 6.0 |
| (4) Stearic acid | 1.0 |
| (5) Glycerol | 10.0 |
| (6) Purified water | 65.0 |

Table 4

| Emulsion | Component (1) | Component (2) |
|---|---|---|
| A | Saturated fatty acid ($C_8$-$C_{12}$) triglyceride | Glycerol monostearate |
| B | Liquid paraffin | Propylene glycol monostearate |
| C | Isopropyl palmitate | Glycerol monostearate |
| F | Refined avocado oil | Glycerol monostearate |

The partially hydrogenated egg yolk lecithin had a phospholipid content of 25%. Each emulsion was prepared by mixing components (1), (2), (3) and (4), heating the mixture to obtain a solution, adding a solution prepared previously by heating a mixture of components (5) and (6) to 75°C to the former solution maintained at 75°C under stirring to obtain an emulsion and cooling the same to 35°C to obtain the sample.

Method and results:

Affinity of each sample for the skin was examined to obtain excellent results and the sample produced a mild, comfortable feeling upon use. Changes after the storage at 40°C were examined to reveal that the appearance was unchanged and stable even after storage for one month in all cases.

The following examples will further illustrate the present invention.

Example 1 (O/W-type moisture cream)

Recipe:

| | Components | Part by weight |
|---|---|---|
| (1) | Squalane | 7.0 |
| | Saturated fatty acid ($C_i$—$C_{12}$ triglyceride | 15.0 |
| | Stearic acid | 3.0 |
| | Cholesterol | 0.5 |
| | Partially hydrogenated egg yolk lecithin | 5.0 |
| | Sucrose stearate | 2.0 |
| | Glycerol stearate | 4.0 |
| | Polypeptide oleate | 0.5 |
| | Propyl p-hydroxybenzoate | 0.1 |
| (2) | Methyl p-hydroxybenzoate | 0.1 |
| (3) | Propylene glycol | 8.0 |
| (4) | Perfume | 0.1 |
| (5) | Purified water | 54.7 |
| | Total | 100.7 |

Components of group (1) were heated to obtain a homogeneous solution. The solution was maintained at 70°C. A homogeneous solution prepared previously by heating components (2), (3) and (5) to said temperature was added to the former solution under stirring to effect emulsification. The resulting emulsion was cooled under stirring and then component (4) was added thereto at 40°C. The mixture was cooled under stirring to obtain a moisture cream. It had a sufficient stability and mild effects on the skin and produced a comfortable feeling upon use. The partially hydrogenated egg yolk lecithin had a phospholipid content of 30%.

Example 2 (O/W-type emulsion)

Recipe:

| | Components | Part by weight |
|---|---|---|
| (1) | Isopropyl palmitate | 4.0 |
| | Avocado oil | 0.5 |
| | Straight-chain fatty acid ($C_8$—$C_{12}$ triglyceride | 3.5 |
| | Partially hydrogenated egg yolk lecithin | 3.0 |
| | Stearic acid | 1.0 |
| | Glycerol monostearate | 2.0 |
| | Propylene glycol monostearate | 0.5 |
| (2) | Glycerol | 10.0 |
| (3) | Methylparaben | 0.2 |
| (4) | Algae colloid | 0.2 |
| (5) | Perfume | 0.05 |
| (6) | Purified water | 74.05 |
| | Total | 100.0 |

Components of group (1) were heated to obtain a homogeneous solution. The solution was maintained at 70°C. A homogeneous solution prepared previously by heating components (2), (3), (4) and (6) to said temperature was added to the former solution under stirring to effect emulsification. The resulting emulsion was cooled under stirring and then component (5) was added thereto at 40°C. The mixture was cooled under stirring to obtain a cosmetic emulsion. The cosmetic emulsion had a sufficient stability and mild effects upon the skin and produced a comfortable feeling upon use. The partially hydrogenated egg yolk lecithin had a phospholipid content of 65%.

Example 3 (Hydrophilic ointment)

Recipe:

| | Components | Part by weight [I] | Part by weight [II] |
|---|---|---|---|
| (1) | Stearyl alcohol | 5.0 | 12.0 |
| (1) | White petrolatum | 5.0 | 20.0 |
| (1) | Liquid paraffin | 10.0 | 10.0 |
| (1) | Partially hydrogenated egg yolk lecithin | 2.0 | 1.0 |
| (1) | Glycerol monostearate | 5.0 | 3.0 |
| (1) | Sucrose stearate | 3.0 | — |
| (1) | Decaglycerol monomyristate | — | 3.0 |
| (2) | Propylene glycol | 10.0 | 12.0 |
| (3) | Methyl p-hydoxybenzoate | 0.15 | 0.05 |
| (4) | Ethyl p-hydroxybenzoate | 0.05 | 0.05 |
| (5) | Purified water | 59.8 | 38.9 |
| | Total | 100.0 | 100.0 |

Components of group (1) were heated to obtain a homogeneous solution. Another homogeneous solution prepared previously by heating components (2), (3), (4) and (5) to said temperature was added to the former solution under stirring to effect emulsification. The resulting emulsion was cooled under stirring to obtain hydrophilic ointments [I] and [II]. They had a sufficient stability and mild effects on the skin and produced a comfortable feeling upon use. The partially hydrogenated egg yolk lecithin had a phospholipid content of 25%.

## Claims

1. An emulsion-type composition for external use containing an effective amount of lecithin for use on the skin, and a fatty acid mono-ester of glycerine or propylene glycol effective as an emulsifier, characterized in that the lecithin is a partially hydrogenated egg yolk lecithin, and in, that it contains from 0.2 to 10 parts by weight per 1 part by weight of lecithin of said fatty acid mono-ester of glycerine or propylene glycol.

2. An emulsion-type composition according to claim 1, characterized in that the fatty acid mono-ester of glycerine or propylene glycol is an ester with a fatty acid having 8 to 32 carbon atoms.

3. The use of the emulsion-type composition according to claim 1 or 2 to prepare a medicine for external use or a cosmetic.

## Patentansprüche

1. Mittel vom Emulsiontyp zur äußerlichen Anwendung, das eine wirksame Menge an Lecithin zur Anwendung auf der Haut und einen Fettsäuremonoester von Glycerin oder Propylenglycol als Emulgiermittel enthält, dadurch gekennzeichnet, daß das Lecithin teilweise hydriertes Lecithin aus Eigelb ist, und dadurch, daß sie 0,2 bis 10 Gewichtsteile des Fettsäuremonoesters von Glycerin oder Propylenglycol pro 1 Gewichtsteil Lecithin enthält.

2. Mittel vom Emulsionstyp nach Anspruch 1, dadurch gekennzeichnet, daß der Fettsäuremonoester von Glycerin oder Propylenglycol ein Ester mit einer Fettsäure mit 8 bis 32 Kohlenstoffatomen ist.

3. Verwendung des Mittels vom Emulsionstyp nach Anspruch 1 oder 2 zur Herstellung eines Arzneimittels zur äußeren Anwendung oder eines Kosmetikums.

**Revendications**

1. Composition de type émulsion pour l'utilisation par voie externe, contenant une quantité efficace de lécithine pour l'application à la peau, et un monoester d'acide gras de glycérol ou de propylèneglycol efficace comme émulsifiant, caractérisée en ce que la lécithine est une lécithine dérivée de jaune d'oeuf partiellement hydrogénée, et en ce qu'elle contient de 0,2 à 10 parties en poids pour une partie en poids de lécithine dudit monoester d'acide gras du glycérol ou du propylèneglycol.

2. Composition de type émulsion selon la revendication 1, caractérisée en ce que le monester d'acide gras du glycérol ou du propylèneglycol est un ester d'un acide gras ayant 8 à 32 atomes de carbone.

3. Utilisation de la composition de type émulsion selon la revendication 1 à 2 pour préparer un médicament pour l'utilisation par voie externe ou un cosmétique.